# EUROPEAN PATENT APPLICATION

(11) **EP 4 789 644 A1**
(43) Date of publication of application: **12.08.2026**
(21) Application number: 25832267.6
(22) Date of filing: 27.06.2025
(51) Int. Cl.: A61F 9/00, A61J 1/05, A61J 1/14, B65D 47/18, B65D 47/40

(54) **GAS-BLOCKING STERILE DRIP BOTTLE CAPABLE OF EXTERNALLY TREATING RESIDUAL LIQUID**

(30) Priority: 03.07.2024 CN 202410886224
(71) Applicant: ZHANGJIAGANG ZHONGHUI MEDICAL PLASTIC TECHNOLOGY CO., LTD., Jiangsu 215600 (CN)
(72) Inventor: WANG, Hui, Suzhou, Jiangsu 215600 (CN); XU, Yan, Suzhou, Jiangsu 215600 (CN)
(74) Representative: Chung, Hoi Kan
(86) International application number: PCT/CN2025/104578
(87) International publication number: WO 2026/007841

(57) **Abstract**

The present disclosure discloses a gas-blocking sterile dropper bottle for externally treating residual liquid, including: a drip head, a bottle body, and a bottle cap. The drip head comprises a drip nozzle, a valve body, a diaphragm valve, and a sterilizing filter. A liquid returning groove, an air returning hole, a gas collection groove, a liquid inlet hole and a plurality of isolation grooves are provided on the valve body. A hollow spring column is provided in the gas collection groove. The sterilizing filter is sealed on the valve body. The diaphragm valve is arranged to blocked on the gas collection groove. A return spring is provided between the diaphragm valve and the hollow spring column. A conical valve core is provided on the diaphragm valve. A drip nozzle is hermetically snap-fitted to the valve body and presses against the diaphragm valve. A drip column is provided on the drip nozzle. A conical drip hole is provided in the drip column. A distributor is sleeved on the drip nozzle, the distributor being sealed against the drip nozzle. A plurality of drainage grooves are circumferentially and evenly arranged on the distributor. The distributor is sealed against the valve body. The present disclosure can prevent outside air from contaminating the conical drip hole and ensure that the bottle body has good reset capability.

## Description

### TECHNICAL FIELD

The present disclosure relates to the field of medical packaging, and in particular to a gas-blocking sterile dropper bottle for externally treating residual liquid.

### BACKGROUND

In daily life, it is necessary to use dropper bottles for precise drug administration. The most common one is the eye dropper bottle. Current dropper bottles on the market have problems such as the liquid medicine being contaminated by air and then flowing back into the bottle, and residual liquid medicine remaining on the drip head after dripping. To address these issues, various structures of dropper bottles or drip heads have appeared on the market. However, these dropper bottles or drip heads on the market all have certain drawbacks. For example, during backflow of air and residual liquid medicine, the residual liquid medicine clogs the sterilizing filter with hydrophobic characteristic, thus affecting the reset of the bottle body; or, the residual liquid medicine remains in the drip hole, and during subsequent dripping, the liquid medicine in the bottle body mixes with the residual liquid medicine, becoming contaminated and affecting the medication efficacy; or, the residual liquid medicine remains on the diaphragm valve and contacts the return spring, thereby corroding it. The corrosive liquid precipitated after the return spring corrodes then contacts the liquid medicine in the bottle body, causing even more serious harm.

To prevent the above situations, a sterile drip head for pre-processing return air and residual liquid medicine, with Publication No. CN118001152B, has been disclosed on the market. It comprises a separator hermetically sleeved in the drip nozzle. A first relief hole is provided on a top wall of the separator, and a movable gap is provided between the first relief hole and the drip column. An air passing gap is provided between the separator and the dispensing nozzle, and the top outer wall of the separator is sealed against the top inner wall of the dispensing nozzle. A plurality of drainage grooves in communication with the first relief hole and the air-passing gap are provided on the top outer wall of the separator. A return spring is provided between the separator and the diaphragm valve. When air and residual liquid flow back together, they enter the air passing gap and the annular air flow channel through the drainage grooves on the separator. The residual liquid and air are separated; the residual liquid is retained in the isolation groove, while the air flows back into the bottle body through the sterilizing filter. This prevents the residual liquid medicine from clogging the sterilizing filter and corroding the return spring. After a period of use, it was found that this structure also has certain drawbacks. Since the residual liquid mostly remains in the drip hole, and the outside air needs to pass through the drip hole with a small diameter before it can flow back into the bottle body, this leads to the situation that when the liquid volume in the bottle body is in a large amount, requiring a small amount of air for backflow compensation, the kinetic energy generated by the air volume entering the drip hole is insufficient to drive the residual liquid medicine in the drip hole to flow back, making backflow blockage more likely. When the liquid volume in the bottle body is in a relatively small amount, requiring a large amount of air for backflow compensation, it takes a long time to complete the backflow compensation. Furthermore, when the drip head is not covered by the cap, the outside air directly contacts the drip hole and contaminates it.

### SUMMARY

The object of the present disclosure is to provide a gas-blocking sterile dropper bottle for externally treating residual liquid, which can reduce the risk of contamination of the drip hole by outside air and ensure the rate of air backflow compensation into the bottle body during separation of air and residual liquid medicine.

To achieve the foregoing objective, a technical solution used in present disclosure is: a gas-blocking sterile dropper bottle for externally treating residual liquid, comprising: a drip head, a bottle body, and a bottle cap; the drip head being sealed onto the bottle body; the drip head being covered by the bottle cap, wherein the drip head comprises a drip nozzle, a valve body, a diaphragm valve, and a hydrophobic sterilizing filter; a plurality of interconnected isolation grooves are circumferentially and evenly arranged on an outer sidewall of the valve body, and a liquid returning groove in communication with any of the isolation grooves is further provided on the outer sidewall of the valve body; a gas collection groove is formed in the valve body; an air returning hole in communication with the gas collection groove and any of the isolation grooves is provided on the valve body; a hollow spring column is provided on a bottom inner wall of the gas collection groove; the sterilizing filter in communication with the hollow spring column is sealed to a bottom wall of the valve body; a liquid inlet hole is formed in the valve body; the diaphragm valve is provided on the valve body; the diaphragm valve is made of elastic rubber material and blocks the gas collection groove; a spring groove is provided at a bottom of the diaphragm valve; a return spring is provided between the spring groove and the hollow spring column; a conical valve core extends upwardly from the top of the diaphragm valve; the drip nozzle is hermetically snap-fitted to the valve body; the outer diameter of the drip nozzle is smaller than the outer diameter of the valve body; a plurality of pressing blocks are circumferentially and evenly arranged on the drip nozzle, and the pressing blocks are pressed against the diaphragm valve; a liquid inlet gap in communication with the liquid inlet hole is formed among the drip nozzle, the diaphragm valve and the valve body; a drip column is extended from the drip nozzle; a conical drip hole matched with the conical valve core is provided in the drip column; a distributor is sleeved on the drip nozzle; an inner top wall of the distributor is sealed against the drip nozzle; a liquid returning column is provided on the distributor; a drainage hole is provided in the liquid returning column and is sealed against the drip column; a plurality of drainage grooves are circumferentially and evenly arranged between the drainage hole and the inner top wall of the distributor; an inner sidewall of the distributor is sealed against the outer sidewall of the valve body; an annular gap in communication with the drainage grooves and the liquid returning groove is formed between the distributor and the drip nozzle; and the distributor, in cooperation with the valve body, is sealed to the bottle body.

Further, the gas-blocking sterile dropper bottle for externally treating residual liquid, wherein a distributor is sleeved on the drip nozzle, an inner top wall of the distributor is sealed against the drip nozzle, a liquid returning column is provided on the distributor, a drainage hole is provided in the liquid returning column, the drainage hole is sealed against the drip column, a plurality of drainage grooves are circumferentially and evenly arranged between the drainage hole and the inner top wall of the distributor; an inner sidewall of the distributor is sealed against the outer sidewall of the valve body, an annular gap in communication with the drainage grooves and the liquid returning groove is formed between the distributor and the drip nozzle, the distributor, in cooperation with the valve body, is sealed to the bottle body.

Further, the gas-blocking sterile dropper bottle for externally treating residual liquid, wherein an arcuate protrusion is protruded downwardly from an inner top wall of the bottle cap, a recessed portion is recessed upwardly in a bottom wall of the arcuate protrusion, and a plurality of hollow holes are circumferentially and evenly arranged on a top wall of the bottle cap, when the drip head is covered by the bottle cap, the arcuate top walls of the conical valve core and the drip column are covered by the recessed portion of the arcuate protrusion.

Further, the gas-blocking sterile dropper bottle for externally treating residual liquid, wherein mounting steps are recessed upwardly on the bottom wall of the valve body and extend into the gas collection groove; the hollow spring column is provided on the mounting steps and is coaxial with the spring groove; a top of the hollow spring column is higher than the air returning hole; a mounting hole in communication with the hollow spring column is formed in the mounting steps, and the sterilizing filter is hermetically fastened in the mounting hole.

Further, the gas-blocking sterile dropper bottle for externally treating residual liquid, wherein a specific connection structure between the diaphragm valve and the valve body is as follows: a sealing sleeve is provided downwardly on the diaphragm valve; a first sealing ring protrudes on an outer sidewall of the sealing sleeve; an annular step is provided between a sidewall of the diaphragm valve and the sealing sleeve; an annular groove is formed on an inner sidewall of the gas collection groove; the sealing sleeve on the diaphragm valve extends into the gas collection groove; the sealing sleeve is located above the air returning hole; the first sealing ring on the sealing sleeve is sealed against the inner sidewall of the gas collection groove; and the annular step on the diaphragm valve is snap-fitted into the annular groove.

Further, the gas-blocking sterile dropper bottle for externally treating residual liquid, wherein a specific connection structure between the drip nozzle and the valve body is as follows: an annular snap groove is provided on a top wall of the valve body; a positioning groove is provided on one sidewall of the annular snap groove; a connecting snap ring is provided downwardly on the drip nozzle; a side snap ring protrudes on one sidewall of the connecting snap ring; a second sealing ring protrudes on another sidewall of the connecting snap ring; the connecting snap ring on the drip nozzle is extended into the annular snap groove; the side snap ring on the connecting snap ring is snap-fitted into the positioning groove of the annular snap groove, and the second sealing ring on the connecting snap ring is sealed against the other sidewall of the annular snap groove.

Further, the gas-blocking sterile dropper bottle for externally treating residual liquid, wherein twelve isolation grooves are formed on the outer sidewall of the valve body at intervals, the isolation grooves are arranged in four columns, and each column comprises three rows of isolation grooves; a transverse plate is provided between two adjacent rows of isolation grooves, and a longitudinal plate is provided between two adjacent columns of isolation grooves; the liquid returning groove is provided on any one of the longitudinal plates and in communication with two isolation grooves in an upper row adjacent to the longitudinal plates directly; the air returning hole is provided on the longitudinal plate opposite to the liquid returning groove, and a transition groove in communication with two isolation grooves in a lower row adjacent to the longitudinal plate directly is formed on the longitudinal plate, the transition groove in communication with the air returning hole; three first vent grooves are provided on the other two longitudinal plates at intervals from top to bottom, and the three first vent grooves respectively directly communicate with two isolation grooves left and right adjacent thereto; second vent grooves are provided on the transverse plates between the isolation grooves located in the upper row that do not directly communicate with the liquid returning groove and the isolation grooves in a middle row in the same column; the second vent grooves are in communication with the isolation grooves located in the same column and in the upper row and the middle row; third vent grooves are provided on the transverse plates located below and in the same column as the transverse plates that are not formed with the second vent groove; the third vent groove is in communication with the isolation grooves located in the middle row and the lower row and in the same column.

Further, the gas-blocking sterile dropper bottle for externally treating residual liquid, wherein a third sealing ring protrudes on an inner wall of an upper end of the conical drip hole, and the conical valve core is extended into the conical drip hole and is sealed against the third sealing ring.

Further, the gas-blocking sterile dropper bottle for externally treating residual liquid, wherein a connection structure between the distributor and the valve body and the bottle body is as follows: a positioning ring sleeve extends downwardly from the bottom wall of the valve body, a first S-shaped ring groove is provided on an outer sidewall of the positioning ring sleeve; a second S-shaped ring groove is provided on an inner sidewall of the distributor; an annular bent plate extends outward from the outer sidewall of the distributor, a protruding ring is provided on an inner sidewall of the annular bent plate; a conical snap ring extends from a bottle mouth of the bottle body, a shoulder is provided between the conical snap ring and an outer sidewall of the bottle mouth; the first S-shaped ring groove on the positioning ring sleeve and the second S-shaped ring groove on the distributor are interlocked and snap-fitted with each other; the distributor and the valve body are hermetically assembled together; the positioning ring sleeve is snap-fitted into the bottle mouth; the distributor is snap-fitted into an inner sidewall of the conical snap ring, and the protruding ring on the annular bent plate is snap-fitted with the shoulder in an offset manner.

The advantages of the present disclosure are that after dripping is completed, the conical valve core on the diaphragm valve can block the conical drip hole in the drip nozzle under the action of the return spring to keep outside air and residual liquid medicine out together, so that outside air cannot enter the conical drip hole to contaminate it. Furthermore, during backflow of the residual liquid medicine, the drainage grooves are directly communicated with the external air, thus avoiding the problems of backflow blockage or excessively long bottle body reset time caused by the limitation of the drip hole diameter as in CN118001152B, thereby ensuring that the bottle body has good reset performance. By configuring the top walls of the drip column and the conical valve core as upwardly convex arcuate walls, and the top wall of the conical valve core not being lower than the top wall of the drip column, and configuring the top wall of the liquid returning column as a downwardly concave arcuate wall, when residual liquid medicine remains on the top walls of the drip column and the conical valve core, it can automatically flow into the drainage grooves by gravity.

### BRIEF DESCRIPTION OF THE DRAWINGS

FIG.1 is a schematic diagram of a gas-blocking sterile dropper bottle for externally treating residual liquid according to the present disclosure.
FIG.2 is a schematic diagram of a drip head of FIG.1.
FIG.3 is a schematic diagram of a valve body of FIG.2.
FIG.4 is a schematic diagram of the valve body of FIG.2 from another perspective.
FIG.5 is a sectional diagram of the valve body of FIG.2.
FIG.6 is a schematic diagram of a drip nozzle of FIG.2.
FIG.7 is a schematic diagram of a diaphragm valve of FIG.2.
FIG.8 is a schematic diagram of a distributor of FIG.2.
FIG.9 is a schematic diagram of a bottle body of FIG.1.
FIG.10 is a schematic diagram of a bottle cap of FIG.1.

### Detailed Description

The solution of the present disclosure will be further described below with reference to the drawings and specific embodiments.

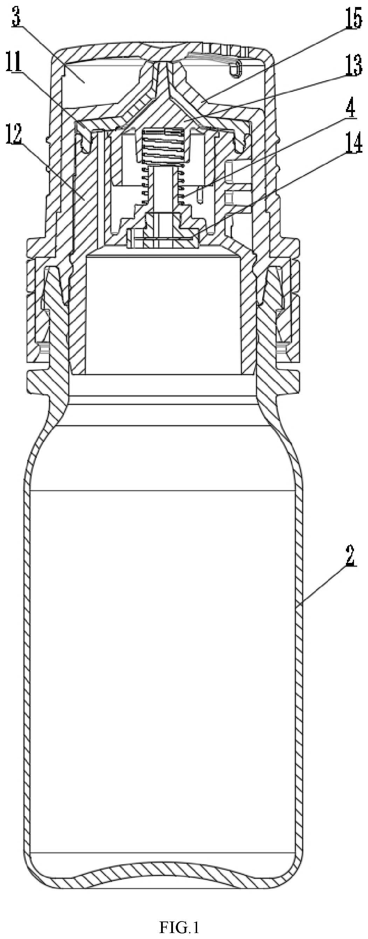

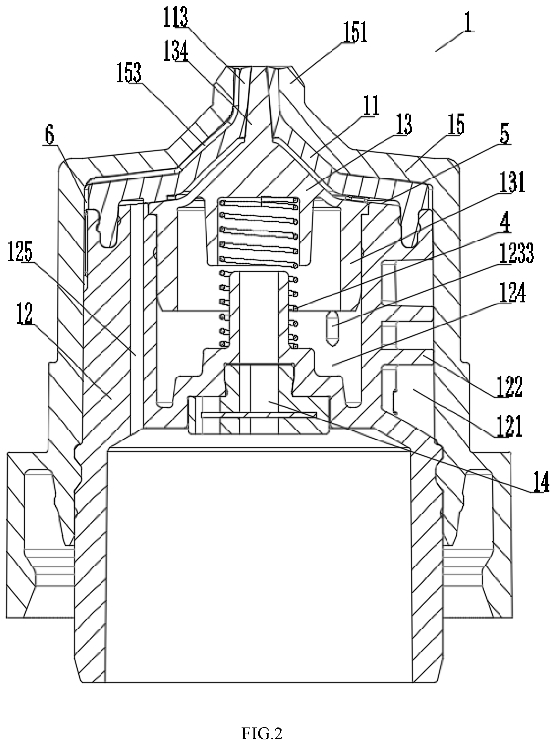

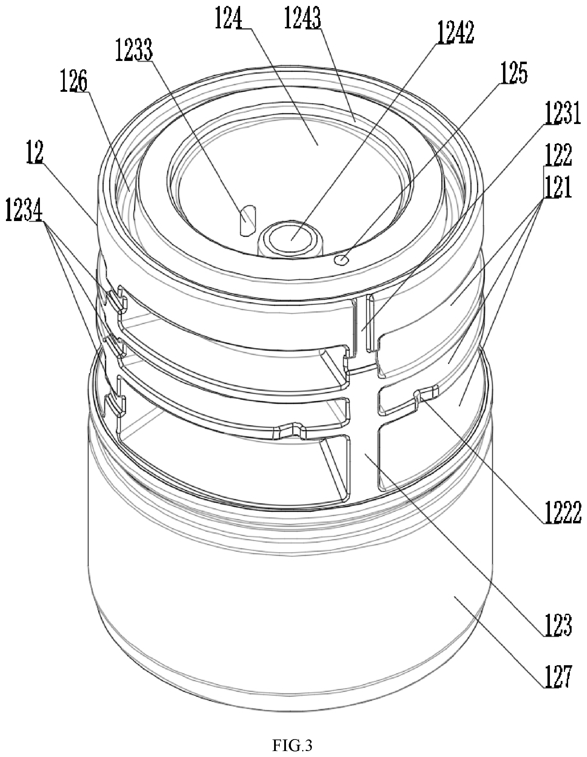

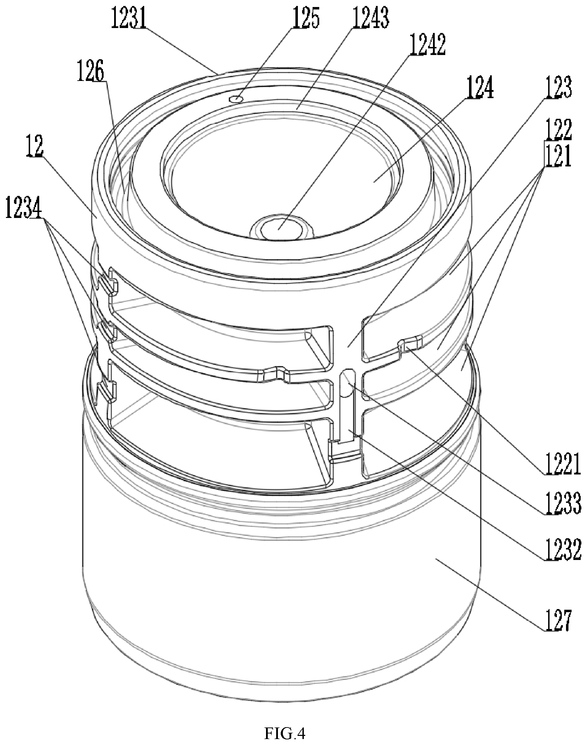

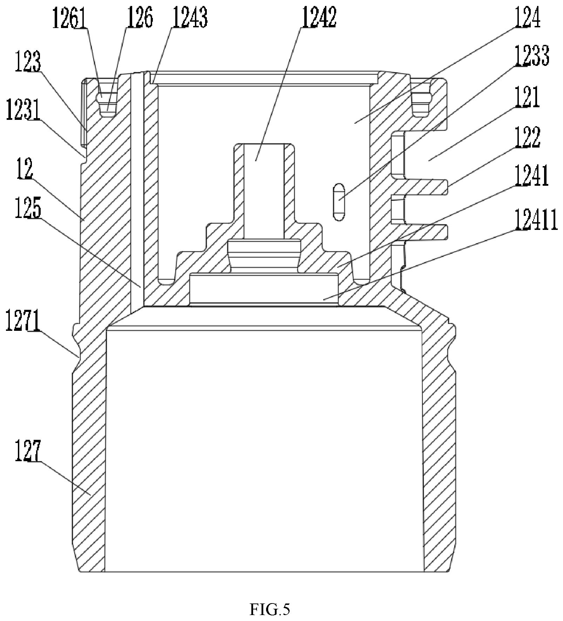

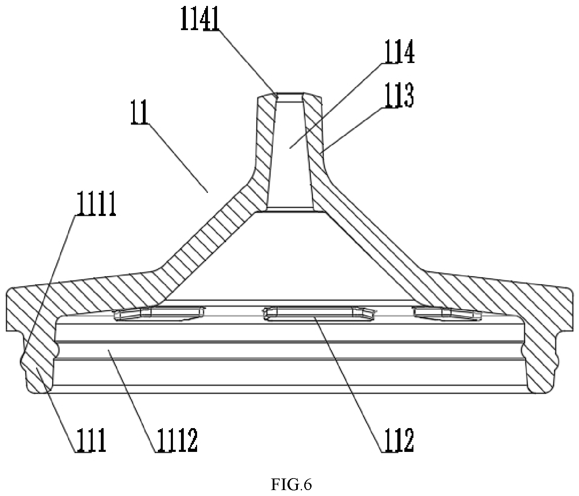

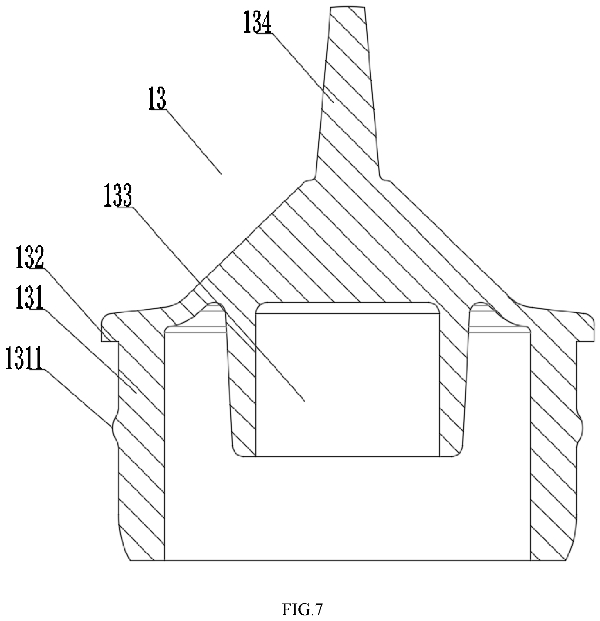

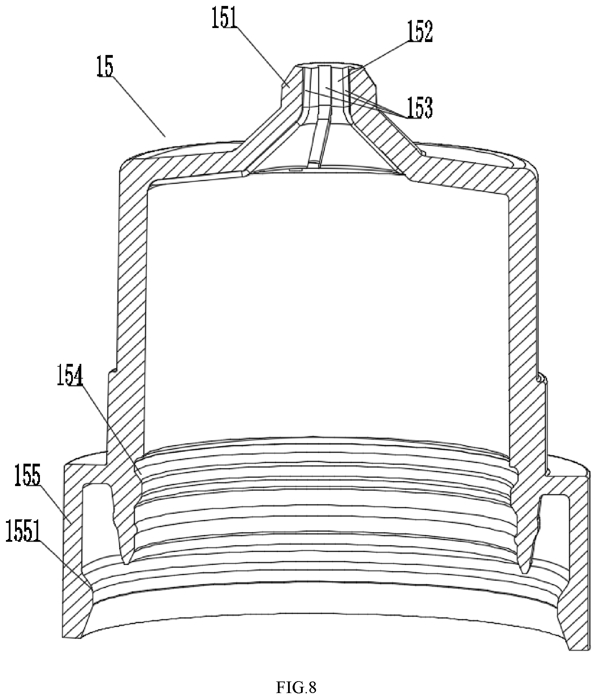

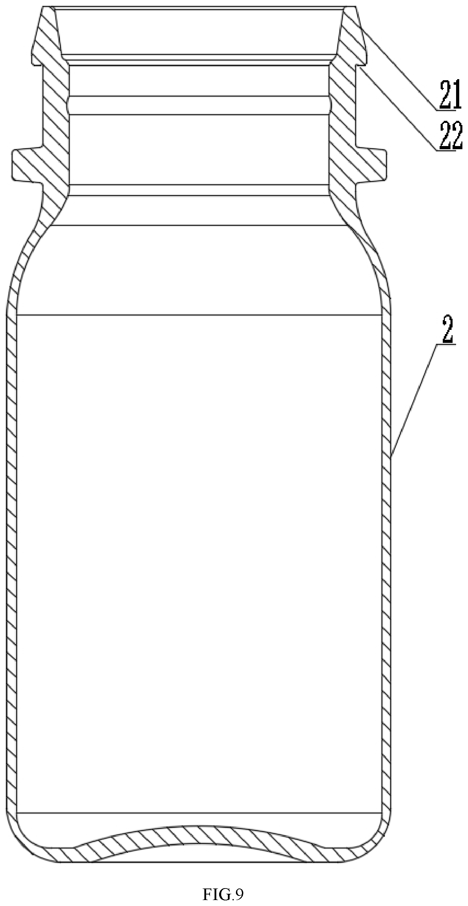

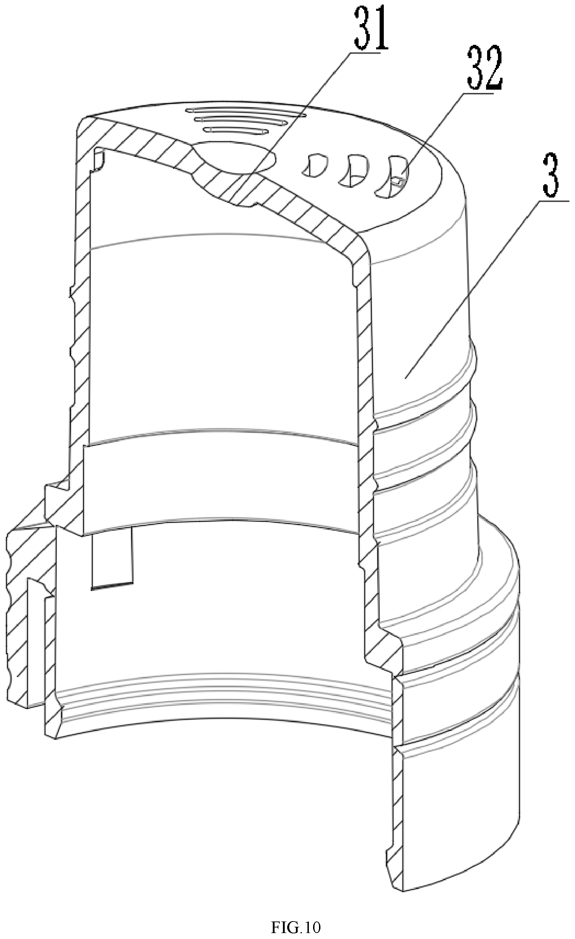

As shown in FIG.1 to FIG.10, a gas-blocking sterile dropper bottle for externally treating residual liquid according to the present disclosure, comprises a drip head 1, a bottle body 2, and a bottle cap 3. The drip head 1 is sealed onto the bottle body 2. The drip head 1 is covered by the bottle cap 3.

The drip head 1 comprises a drip nozzle 11, a valve body 12, a diaphragm valve 13, and a sterilizing filter 14 with hydrophobic characteristic. Twelve isolation grooves 121 are formed on the outer sidewall of the valve body 12 at intervals, the isolation grooves are arranged in four columns, and each column comprises three rows of isolation grooves; a transverse plate 122 is provided between two upwardly and downwardly adjacent rows of isolation grooves 121, and a longitudinal plate 123 is provided between two adjacent columns of isolation grooves. Liquid returning groove 1231 is provided on any one of the longitudinal plates 123, and the liquid returning groove 1231 directly communicates with two isolation grooves 121 in an upper row adjacent to the longitudinal plates 123. A transition groove 1232 is formed on the longitudinal plate 123 opposite to the liquid returning groove 1231, and the transition groove 1232 directly communicates with two isolation grooves 121 in a lower row adjacent to the longitudinal plate 123. An air returning hole 1233 is provided in the transition groove 1232. Three first vent grooves 1234 are provided on the other two longitudinal plates 123 at intervals from top to bottom. The three first vent grooves 1234 respectively directly communicate with two isolation grooves 121 left and right adjacent to each of said three first vent grooves. second vent grooves 1221 are provided on the transverse plates 122 between the isolation grooves 121 in the upper row that do not directly communicate with the liquid returning groove 1231 and the isolation groove 121 located in a middle row located in the same column as said isolation groove, the second vent grooves 1221 in communication with the isolation grooves 121 located in the same column with said isolation grooves and in the upper and middle row; third vent groove 1222 are provided on the transverse plates 122 in the same column as the transverse plates 122 that are not formed with second vent grooves 1221 above said transverse plates, the third vent groove 1222 is in communication with the isolation grooves 121 located in the middle row and the lower row and in the same column as said isolation grooves. After the gas enters, through the liquid returning groove 1231, the two isolation grooves 121 in the upper row adjacent to said liquid returning groove, the gas then enters, through the first vent grooves 1234 in the upper row, the other two isolation grooves 121 in the upper row that are remote from the liquid returning groove 1231 and not directly communicated with the liquid returning groove 1231. Then, the gas enters, through the second vent grooves 1221 on the transverse plates 122 below said two isolation grooves 121, the isolation grooves 121 in the middle row in the same columns as said two isolation grooves 121. Subsequently, the gas enters, through the first vent grooves 1234 in the middle row that are communicated with said two isolation grooves 121, the other two isolation grooves 121 in the middle row that are close to the liquid returning groove 1231 and remote from the air returning hole 1233. Then, the gas enters, through the third vent grooves 1222 on the transverse plates 122 below said two isolation grooves 121, the isolation grooves 121 in the lower row in the same columns as said two isolation grooves 121. Subsequently, the gas enters, through the first vent grooves 1234 in the lower row that are communicated with said two isolation grooves 121, the other two isolation grooves 121 in the lower row that are close to the air returning hole 1233 and directly communicated with the transition groove 1232. Finally, the gas enters the air returning hole 1233 through the transition groove 1232.

A gas collection groove 124 is formed in the valve body 12, the gas collection groove 124 being in communication with an air returning hole 1233. The air entering the air returning hole 1233 is able to enter the gas collection groove 124. Mounting steps 1241 are recessed upwardly on the bottom wall of the valve body 12, the mounting steps 1241 extending upwardly into the gas collection groove 124. A mounting hole 12411 is formed in the mounting steps 1241, and the sterilizing filter 14 is hermetically fastened in the mounting hole 12411. A hollow spring column 1242 in communication with the mounting hole 12411 is vertically provided on the mounting steps 1241. The air in the gas collection groove 124 passes through the hollow spring column 1242 and then passes through the sterilizing filter 14. The top of the hollow spring column 1242 is higher than the air returning hole 1233. A liquid inlet hole 125 throughout the top wall and the bottom wall of the valve body 12 is formed in the valve body 12.

The diaphragm valve 13 with good elasticity is provided on the valve body 12. A sealing sleeve 131 is provided downwardly on the diaphragm valve 13. A first sealing ring 1311 protrudes on an outer sidewall of the sealing sleeve 131. An annular step 132 is provided between a sidewall of the diaphragm valve 13 and the sealing sleeve 131. An annular groove 1243 is formed on an inner sidewall of the gas collection groove 124. The sealing sleeve 131 on the diaphragm valve 13 extends into the gas collection groove 124, and the sealing sleeve 131 is located above the air returning hole 1233, and the first sealing ring 1311 on the sealing sleeve 131 is sealed against the inner sidewall of the gas collection groove 124, and the annular step 132 on the diaphragm valve 13 is snap-fitted into the annular groove 1243. The diaphragm valve 13 blocks the gas collection groove 124. A spring groove 133 coaxial with the hollow spring column 1242 is provided at a bottom of the diaphragm valve 13. A return spring 4 is provided between the spring groove 133 and the hollow spring column 1242. A conical valve core 134 coaxial with the spring groove 133 is extended upwardly from a top of the diaphragm valve 13.

The drip nozzle 11 is hermetically snap-fitted to the valve body 12. An annular snap groove 126 is provided on a top wall of the valve body 12. A positioning groove 1261 is provided on one sidewall of the annular snap groove 126. A connecting snap ring 111 is provided downwardly on the drip nozzle 11. A side snap ring 1111 protrudes on one sidewall of the connecting snap ring 111, and a second sealing ring 1112 protrudes on another side wall of the connecting snap ring 111. The connecting snap ring 111 on the drip nozzle 11 is extended into the annular snap groove 126, and the side snap ring 1111 on the connecting snap ring 111 is snap-fitted into the positioning groove 1261 of the annular snap groove 126, and the second sealing ring 1112 on the connecting snap ring 111 is sealed against the other sidewall of the annular snap groove 126. A plurality of pressing blocks 112 are circumferentially and evenly arranged on the drip nozzle 11, and the pressing blocks 112 are pressed against the diaphragm valve 13. A liquid inlet gap 5 in communication with the liquid inlet hole 125 is formed among the drip nozzle 11, the diaphragm valve 13 and the valve body 12. An outer diameter of the drip nozzle 11 is smaller than an outer diameter of the valve body 12. A drip column 113 is extended from the drip nozzle 11. A conical drip hole 114 matched with the conical valve core 134 is provided in the drip column 113. A third sealing ring 1141 protrudes on an inner wall of an upper end of the conical drip hole 114. Under the elastic force of the return spring 4, the diaphragm valve 13 will cause the conical valve core 134 to be extended into the conical drip hole 114 and the conical valve core 134 will be sealed against the third sealing ring 1141.

A distributor 15 is sleeved on the drip nozzle 11, and an inner top wall of the distributor 15 is sealed against the drip nozzle 11. A liquid returning column 151 is provided on the distributor 15. A drainage hole 152 is provided in the liquid returning column 151, the drainage hole 152 being sealed against the drip column 113. A plurality of drainage grooves 153 are circumferentially and evenly arranged between the drainage hole 152 and the inner top wall of the distributor 15. An inner sidewall of the distributor 15 is sealed against the outer sidewall of the valve body 12. An annular gap 6 in communication with the drainage grooves 153 and the liquid returning groove 1231 is formed between an inner sidewall of the distributor 15 and an outer sidewall of the drip nozzle 11. The drip column 113 and the conical valve core 134 each have an upwardly convex arcuate top wall, and the top wall of the conical valve core 134 is not lower than the top wall of the drip column 113, and the liquid returning column 151 has a downwardly concave arcuate top wall.

The distributor 15, in cooperation with the valve body 12, is sealed to the bottle body 2. A positioning ring sleeve 127 extends downwardly from the bottom wall of the valve body 12. A first S-shaped ring groove 1271 is provided on an outer sidewall of the positioning ring sleeve 127. A second S-shaped ring groove 154 is provided on an inner sidewall of the distributor 15. An annular bent plate 155 extends outward from the outer sidewall of the distributor 15. A protruding ring 1551 is provided on an inner sidewall of the annular bent plate 155. A conical snap ring 21 extends from a bottle mouth of the bottle body 2. A shoulder 22 is provided between the conical snap ring 21 and an outer sidewall of the bottle mouth. The first S-shaped ring groove 1271 on the positioning ring sleeve 127 and the second S-shaped ring groove 154 on the distributor 15 are interlocked and snap-fitted with each other, and the distributor 15 and the valve body 12 are hermetically assembled together, and the positioning ring sleeve 127 is snap-fitted into the bottle mouth, and the distributor 15 is snap-fitted into an inner sidewall of the conical snap ring 21, and the protruding ring 1551 on the annular bent plate 155 is snap-fitted with the shoulder 22 in an offset manner, thereby hermetically assembling the bottle mouth of the bottle body 2 with the valve body 12 and the distributor 15 together.

An arcuate protrusion 31 is protruded downwardly from an inner top wall of the bottle cap 3, and a recessed portion is recessed upwardly in a bottom wall of the arcuate protrusion 31. When the drip head 1 is covered by the bottle cap 3, the bottle cap 3 connecting to the distributor 15, the arcuate top walls of the conical valve core 134 and the drip column 113 are covered by the recessed portion of the arcuate protrusion 31, thereby protecting top walls of the conical valve core 134 and the drip column 113 from outside air contamination. A plurality of hollow holes 32 are circumferentially and evenly arranged on a top wall of the bottle cap. When the drip head 1 is covered by the bottle cap 3, the isolation groove 121 keeps in communication with the outside air, which facilitates the evaporation of residual liquid medicine in the isolation groove 121.

During dripping, the bottle cap 3 is removed, and the dropper bottle is inverted. Due to the hydrophobic characteristic of the sterilizing filter 14, the liquid medicine in the bottle body 2 cannot pass through the sterilizing filter 14 into the gas collection groove 124. The liquid medicine in the bottle body 2 enters the liquid inlet gap 5 through the liquid inlet hole 125. When the bottle body 2 is squeezed, the pressurized liquid medicine in the bottle body 2 passes through the liquid inlet hole 125 into the liquid inlet gap 5 and pushes against the diaphragm valve 13. Since the diaphragm valve 13 is made of an elastic plastic cement material, when the diaphragm valve 13 is pushed by the liquid medicine in the liquid inlet gap 5, the unfixed middle portion of the diaphragm valve 13 overcomes the elastic force of the return spring 4 and then moves away from the drip nozzle 11, at which point, the conical valve core 134 of the diaphragm valve 13 separates from the conical drip hole 114 in the drip column 113 to form a liquid outlet passage. The liquid medicine passes through the liquid outlet passage and forms a drip of liquid medicine on the top walls of the drip column 113 and the conical valve core 134, and then drips downward.

When the bottle body 2 is released, the liquid medicine in the liquid inlet gap 5 no longer pushes against the diaphragm valve 13. The diaphragm valve 13 is reset under the elastic force of the return spring 4 and is resealed against the conical drip hole 114, and the liquid inlet gap 5 is isolated from the outside air. The residual liquid medicine remaining in the conical drip hole 114 is discharged and remains on the top walls of the drip column 113 and the conical valve core 134. Meanwhile, a negative pressure will be generated when the bottle body 2 is reset. Under the effect of the negative pressure, outside air is drawn into the drainage grooves 153. When the dropper bottle is still in an inverted state, because the drainage grooves 153 are circumferentially and evenly arranged, the outside air drawn into each drainage groove 153 forms a relatively strong vortex, which entrains the residual liquid medicine remaining on the top walls of the drip column 113 and the conical valve core 134 into the drainage grooves 153. When the dropper bottle is in an upright state, due to the drip column 113 and the conical valve core 134 each have an upwardly convex arcuate top wall, the residual liquid medicine automatically flows into the drainage grooves 153 by gravity. When negative pressure is present, it acts directly on the residual liquid medicine. After the air and the residual liquid medicine are drawn together into the drainage grooves 153, they then enter the annular gap 6. Due to the small flow space of the drainage grooves 153, the air entering the drainage grooves 153 is compressed, increasing the air pressure and accelerating the flow rate. When the air and residual liquid medicine enter the annular gap 6, due to the large space of the annular gap 6, the air pressure decreases and the flow rate also decreases. A large amount of residual liquid separates from the air by its own weight, and the air and the separated residual liquid medicine flow separately. The air passes through the liquid returning groove 1231, the isolation grooves 121, the first vent grooves 1234, the third vent grooves 1222, the second vent grooves 1221, the transition groove 1232, and the air returning hole 1233, and then enters the gas collection groove 124. Since there are twelve isolation grooves 121, the air undergoes multiple changes in flow direction during backflow, which enhances the collision and adhesion between the residual liquid medicine in the air and the walls of the isolation grooves 121, thereby further improving the separation efficiency of air and residual liquid medicine. As a result, when the air enters the air returning hole 1233, it contains no residual liquid. When the air enters the gas collection groove 124 from the air returning hole 1233, due to the large space of the gas collection groove 124, the flow rate of the air entering the gas collection groove 124 from the air returning hole 1233 is further reduced. Moreover, the top of the hollow spring column 1242 is higher than the air returning hole 1233, so the air coming out of the air returning hole 1233 cannot directly enter the hollow spring column 1242 and come into contact with the sterilizing filter 14. Instead, it fills the gas collection groove 124, forcing the air located above the top of the hollow spring column 1242 to enter the hollow spring column 1242, and finally enters the bottle body 2 after being sterilized by the sterilizing filter 14. This prevents the backflow rate of air from being too fast and damaging the sterilizing filter 14. The residual liquid medicine is isolated in the isolation grooves 121. The residual liquid gradually flows into the isolation grooves 121 in the lower row by its own weight. Moreover, because there are a large number of isolation grooves 121, the residual liquid medicine does not easily flow out of the isolation grooves 121, and thus does not affect subsequent dripping.

Finally, it should be noted that the above embodiments are only used to illustrate the technical scheme of the present disclosure rather than limit it. Although the present disclosure has been described in detail with reference to the above embodiments, those skilled in the art should understand that the specific embodiment of the disclosure can still be modified or equivalent replaced. Any modification or equivalent replacement without departing from the spirit and scope of the disclosure shall be covered within the protection scope of the claims of the disclosure.

## Claims

1. A gas-blocking sterile dropper bottle for externally treating residual liquid, comprising: a drip head (1), a bottle body (2), and a bottle cap (3); the drip head (1) being sealed onto the bottle body (2); the drip head (1) being covered by the bottle cap (3), wherein the drip head (1) comprises a drip nozzle (11), a valve body (12), a diaphragm valve (13), and a hydrophobic sterilizing filter (14); a plurality of interconnected isolation grooves (121) are circumferentially and evenly arranged on an outer sidewall of the valve body (12), and a liquid returning groove (1231) in communication with any of the isolation grooves (121) is further provided on the outer sidewall of the valve body (12); a gas collection groove (124) is formed in the valve body (12); an air returning hole (1233) in communication with the gas collection groove (124) and any of the isolation grooves (121) is provided on the valve body (12); a hollow spring column (1242) is provided on a bottom inner wall of the gas collection groove (124); the sterilizing filter (14) in communication with the hollow spring column (1242) is sealed to a bottom wall of the valve body (12); a liquid inlet hole (125) is formed on the valve body (12); the diaphragm valve (13) is provided on the valve body (12); the diaphragm valve (13) is made of elastic rubber material and blocks the gas collection groove (124); a spring groove (133) is provided at a bottom of the diaphragm valve (13); a return spring (4) is provided between the spring groove (133) and the hollow spring column (1242); a conical valve core (134) extends upwardly from the top of the diaphragm valve (13); the drip nozzle (11) is hermetically snap-fitted to the valve body (12); the outer diameter of the drip nozzle (11) is smaller than the outer diameter of the valve body (12); a plurality of pressing blocks (112) are circumferentially and evenly arranged on the drip nozzle (11), and the pressing blocks (112) are pressed against the diaphragm valve (13); a liquid inlet gap (5) in communication with the liquid inlet hole (125) is formed among the drip nozzle (11), the diaphragm valve (13) and the valve body (12); a drip column (113) is extended from the drip nozzle (11); a conical drip hole (114) matched with the conical valve core (134) is provided in the drip column (113); a distributor (15) is sleeved on the drip nozzle (11); an inner top wall of the distributor (15) is sealed against the drip nozzle (11); a liquid returning column (151) is provided on the distributor (15); a drainage hole (152) is provided in the liquid returning column (151) and is sealed against the drip column (113); a plurality of drainage grooves (153) are circumferentially and evenly arranged between the drainage hole (152) and the inner top wall of the distributor (15); an inner sidewall of the distributor (15) is sealed against the outer sidewall of the valve body (12); an annular gap (6) in communication with the drainage grooves (153) and the liquid returning groove (1231) is formed between the distributor (15) and the drip nozzle (11); and the distributor (15), in cooperation with the valve body (12), is sealed to the bottle body (2).

2. The gas-blocking sterile dropper bottle for externally treating residual liquid of claim 1, wherein the drip column (113) and the conical valve core (134) each have an upwardly convex arcuate top wall, the top wall of the conical valve core (134) is not lower than the top wall of the drip column (113), and the liquid returning column (151) has a downwardly concave arcuate top wall.

3. The gas-blocking sterile dropper bottle for externally treating residual liquid of claim 2, wherein an arcuate protrusion (31) protrudes downwardly from an inner top wall of the bottle cap (3), a recessed portion is recessed upwardly on a bottom wall of the arcuate protrusion (31), a plurality of hollow holes (32) are circumferentially and evenly arranged on a top wall of the bottle cap (3), when the drip head (1) is covered by the bottle cap (3), the arcuate top walls of the conical valve core (134) and the drip column (113) are covered by the recessed portion of the arcuate protrusion (31).

4. The gas-blocking sterile dropper bottle for externally treating residual liquid of claim 1, wherein mounting steps (1241) are recessed upwardly on the bottom wall of the valve body (12) and extend into the gas collection groove (124); the hollow spring column (1242) is provided on the mounting steps (1241) and is coaxial with the spring groove (133); a top of the hollow spring column (1242) is higher than the air returning hole (1233); a mounting hole (12411) in communication with the hollow spring column (1242) is formed in the mounting steps (1241), and the sterilizing filter (14) is hermetically fastened in the mounting hole (12411).

5. The gas-blocking sterile dropper bottle for externally treating residual liquid of claim 1, wherein a sealing sleeve (131) is provided on the diaphragm valve (13); a first sealing ring (1311) protrudes on an outer sidewall of the sealing sleeve (131); an annular step (132) is provided between a sidewall of the diaphragm valve (13) and the sealing sleeve (131); an annular groove (1243) is formed on an inner sidewall of the gas collection groove (124); the sealing sleeve (131) on the diaphragm valve (13) extends into the gas collection groove (124); the sealing sleeve (131) is located above the air returning hole (1233); the first sealing ring (1311) on the sealing sleeve (131) is sealed against the inner sidewall of the gas collection groove (124); and the annular step (132) on the diaphragm valve (13) is snap-fitted into the annular groove (1243).

6. The gas-blocking sterile dropper bottle for externally treating residual liquid of claim 1, wherein an annular snap groove (126) is provided on a top wall of the valve body (12); a positioning groove (1261) is provided on one sidewall of the annular snap groove (126); a connecting snap ring (111) is provided on the drip nozzle (11); a side snap ring (1111) protrudes on one sidewall of the connecting snap ring (111); a second sealing ring (1112) protrudes on another sidewall of the connecting snap ring (111); the connecting snap ring (111) on the drip nozzle (11) is extended into the annular snap groove (126); the side snap ring (1111) on the connecting snap ring (111) is snap-fitted into the positioning groove (1261) of the annular snap groove (126), and the second sealing ring (1112) on the connecting snap ring (111) is sealed against the other sidewall of the annular snap groove (126).

7. The gas-blocking sterile dropper bottle for externally treating residual liquid of claim 1, wherein twelve isolation grooves (121) are formed on the outer sidewall of the valve body (12) at intervals, the isolation grooves (121) are arranged in four columns, and each column comprises three rows of isolation grooves; a transverse plate (122) is provided between two adjacent rows of isolation grooves (121), and a longitudinal plate (123) is provided between two adjacent columns of isolation grooves (121); the liquid returning groove (1231) is provided on any one of the longitudinal plates (123) and in communication with two isolation grooves (121) in an upper row adjacent to the longitudinal plates (123) directly; the air returning hole (1233) is provided on the longitudinal plate (123) opposite to the liquid returning groove (1231), and a transition groove (1232) in communication with two isolation grooves (121) in a lower row adjacent to the longitudinal plate (123) directly is formed on the longitudinal plate (123); the transition groove (1232) is in communication with the air returning hole (1233); three first vent grooves (1234) are provided on the other two longitudinal plates (123) at intervals from top to bottom, and the three first vent grooves (1234) respectively directly communicate with two isolation grooves (121) left and right adjacent thereto; second vent grooves (1221) are provided on the transverse plates (122) between the isolation grooves (121) located in the upper row that do not directly communicate with the liquid returning groove (1231) and the isolation grooves (121) in a middle row in the same column; the second vent grooves (1221) are in communication with the isolation grooves (121) located in the same column and in the upper row and the middle row; third vent grooves (1222) are provided on the transverse plates (122) located below and in the same column as the transverse plates (122) that are not formed with the second vent groove (1221); the third vent groove (1222) is in communication with the isolation grooves (121) located in the middle row and the lower row and in the same column.

8. The gas-blocking sterile dropper bottle for externally treating residual liquid of claim 1, wherein a third sealing ring (1141) is provided on an inner wall of an upper end of the conical drip hole (114), the conical valve core (134) is extended into the conical drip hole (114) and is sealed against the third sealing ring (1141).

9. The gas-blocking sterile dropper bottle for externally treating residual liquid of claim 1, wherein a positioning ring sleeve (127) extends downwardly from the bottom wall of the valve body (12), a first S-shaped ring groove (1271) is provided on an outer sidewall of the positioning ring sleeve (127); a second S-shaped ring groove (154) is provided on an inner sidewall of the distributor (15); an annular bent plate (155) extends outward from the outer sidewall of the distributor (15), a protruding ring (1551) is provided on an inner sidewall of the annular bent plate (155); a conical snap ring (21) extends from a bottle mouth of the bottle body (2), a shoulder (22) is provided between the conical snap ring (21) and an outer sidewall of the bottle mouth; the first S-shaped ring groove (1271) on the positioning ring sleeve (127) and the second S-shaped ring groove (154) on the distributor (15) are interlocked and snap-fitted with each other; the distributor (15) and the valve body (12) are hermetically assembled together; the positioning ring sleeve (127) is snap-fitted into the bottle mouth; the distributor (15) is snap-fitted into an inner sidewall of the conical snap ring (21), and the protruding ring (1551) on the annular bent plate (155) is snap-fitted with the shoulder (22) in an offset manner.
